# EUROPÄISCHE PATENTANMELDUNG

(11) **EP 2 270 378 A1**
(43) Veröffentlichungstag der Anmeldung: **05.01.2011**
(21) Anmeldenummer: 09008699.2
(22) Anmeldetag: 02.07.2009
(51) Int. Cl.: F16L 11/11, F16L 11/15, F16L 51/02, B60H 1/00

(54) **Flexibles Rohr**

(71) Anmelder: LANXESS Deutschland GmbH, 51369 Leverkusen (DE)
(72) Erfinder: Babl, Thomas, Lantau Island Hong Kong (HK); Marbach, Marc, Causeway Bay Hong Kong (HK)

(57) **Zusammenfassung**

Die vorliegende Erfindung betrifft ein flexibles Rohr mit weinigstens einem Faltenbalg der wenigstens eine, bevorzugt mehrere Falten aufweist, die in der Wand des Rohres ausgebildet sind und über einer Außerfläche des Rohres verlaufen, wobei die Falten in axialer Längsrichtung des Rohres nicht ausdrücklich voneinander beabstandet sind und der Faltenbalg wenigstens eine Falte aufweist, die zwei wiederum einander gegenüberliegende Biegezonen und zwei eingeschränkte Verlängerungszonen aufweist, wobei die eingeschränkten Verlängerungszonen zwischen den einander gegenüberliegencien Biegezonen angeordnet sind und die Höhe der Außenfläche der Falten in den eingeschränkten Verlängerungszonen unter der Höhe der Außenfläche der Falter in den Biegezonen aber noch über der Außenfläche des Rohres liegt und die Wanddicke des flexiblen Rohres in mindestens einer eingeschraenkten Verlaeagenmgszone in mindestens einer Falte, bevorzugt in allen eingeschrasniüen Verlaengerungszonen des Rohres, gezielt groesser oder kleiner ausgefuehrt als die Wanddicke in den Biegezonen.

## Beschreibung

Die vorliegende Erfindung betrifft ein flexibles Rohr mit wenigstens einem Faltenbalg der wenigstens eine Falte, bevorzugt mehrere Falten, aufweist, die in der Wand des Rohres ausgebildet sind und über einer Außenfläche des Rohres verlaufen, wobei die Falten in axialer Längsrichtung des Rohres nicht ausdrücklich voneinander beabstandet sind und der Faltenbalg wenigstens eine Falte aufweist, die zwei einander gegenüberliegende Biegezonen und zwei eingeschränkte Veriängerungszonen aufweist, wobei die eingeschränkten Verlängerungszonen zwischen den einander gegenüberliegenden Biegezonen angeordnet sind und die Höhe der Außenfläche der Falten in den eingeschränkten Verlängerungszonen unter der Höhe der Außenfläche der Falten in den Biegezonen, aber noch über der Außenfläche des Rohres liegt und die Wanddicke des flexiblen Rohres in mindestens einer eingeschraenkten Verlaengerungszone in mindestens einer Falte, bevorzugt in allen Falten, der eingeschränkten Verlängerungszonen des Rohres gazielt größer oder kleiner ausgefuehrt als die Wanddicke in den Biegezonen.

DE 197 07 518 C1 betrifft ein Wellrohr aus thermoplastischem Polymermaterial für den Einsatz als Fluidleitung, mit mindestens einer Polymerschicht, aufweisend voneinander in Rohrachsenrichtung beabstandete, geschlossene, geometrische Außenkonturen, die in mindestens einem radialen Winkelbereich in axialer Längsrichtung hintereinander eine Wellung auf dem Rohrmantel definieren, wobei die geschlossenen, geometrischen Außenkonturen so ausgebildet sind, dass zwei einander ungefähr gegenüberliegende Mantellinien der Rohrmantelfläche frei von Wellungen sind und diese Mantellinien sich in Rohrlängsrichtung erstrecken.

WO 1999/0022171 A1 beschreibt einen Faltenbalg aus einem Schlauch mit einem im wesentlichen kreisförmigen Querschnitt, wobei der Schlauch eine Außenfläche und eine Innenfläche aufweist und eine Vielzahl von erhabenen peripheren Falten, die in der Fläche des Schlauches gebildet werden, wobei die Innenfläche des Schlauches die Innenfläche der Falten definiert, und wobei die Außenfläche des Schlauches die Außenfläche der Falten definiert, dadurch gekennzeichnet, dass mindestens eine der Falten so ausgebildet ist, dass ein Abschnitt der Falte im Wesentlichen eben mit der Fläche des Schlauches ist, während der im wesentlichen kreisförmige Querschnitt des Schlauches beibehalten wird.

WO 2002/002981 A1 beschreibt ein flexibles Rohr mit einem Balg, der eine Mehrzahl von Falten aufweist, die in der Wand des Rohrs ausgebildet sind und über einer Außenfläche des Rohrs verlaulen, wobei die Falten in axialer Richtung des Rohrs voneinander bcabstandet sind, wobei wenigstens eine der Falten zwei einander gegenüber liegende Biegesektionen und zwei zwischen den Biegesektionen befindliche eingeschränkte Verlängerungssektionen aufweisen, und wobei die Höhe der Biegesektionen über der Außenfläche des Rohrs größer als die Höhe der eingeschränkten Verlängerungssektionen über der Außenfläche des Rohres ist.

Nachteilig an den Ausführungen des Standes der Technik ist, dass je nach Geometrie die Elastizität der eingeschränkten Velängerungszonen bei bestimmten relativen Bewegungen der beiden Enden des Rohres in axialer Längsrichtung nur durch Änderungen des Werkstoffs bewirkt werden kann. Wenn der Werkstoff vorgegeben ist, kann die Flexibilität des Bauteils nur durch eine Änderung seiner Geometrie eingestellt werden, was wiederum eine Einstellung/Änderung des Werkzeugs mit dem das flexible Rohr oder Wellrohr hergestellt wird, erfordert.

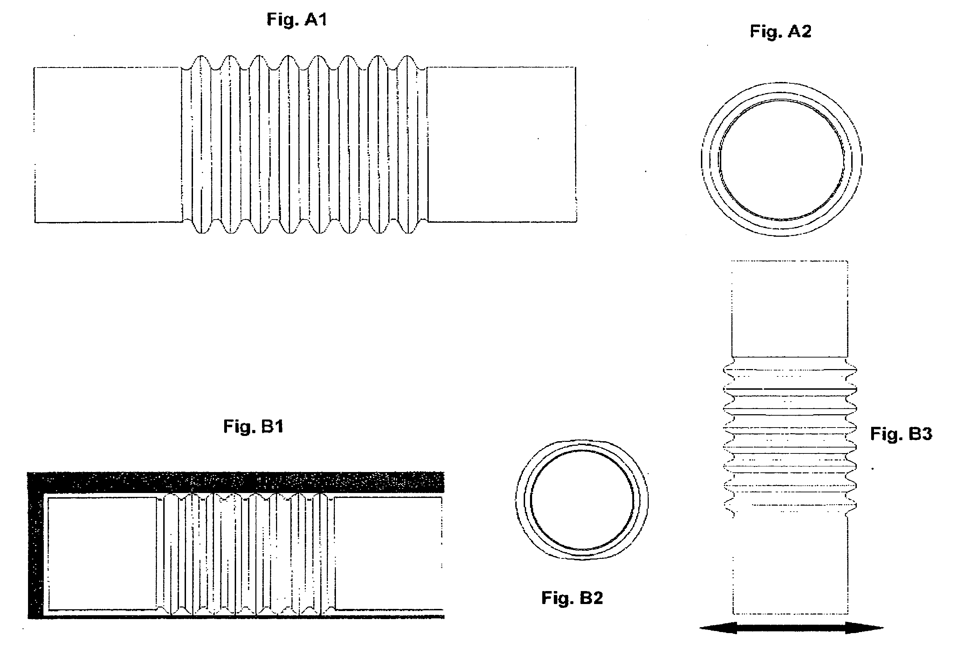

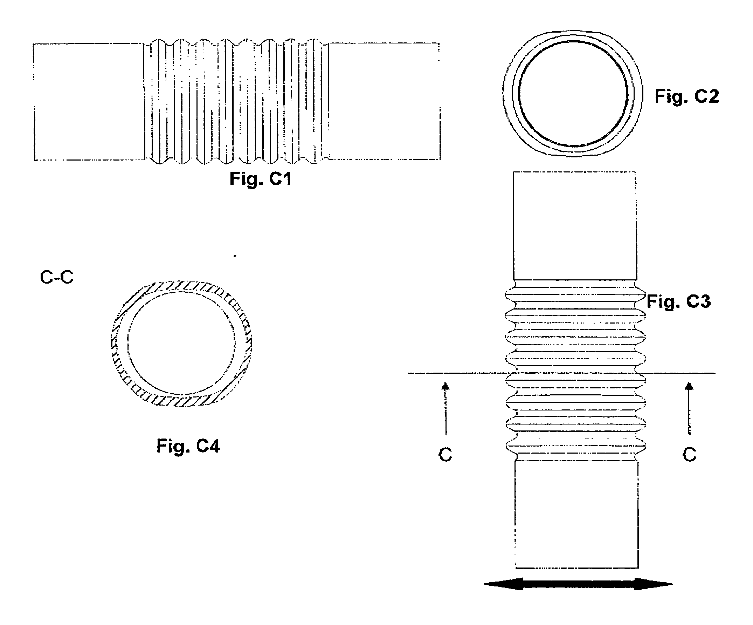

Ferner kann es aufgrund der Änderung der Steifigkeit in den Übergangszonen von den Fallen zum geraden Bereich zwischen zwei Falten bei der Innendruckbeaufschlagung zu Spannungskonzentrationen kommen (siehe z.B. WO 2002/002981A1 Seite 6, Zeilen 8-16) oder die mechanischen Eigenschaften der Komponenten werden durch Einkerbungen negativ beeinflusst (WO 2002/002981 A1, Seite 13, Figuren 6A und 6B).

Aufgrund der Charakteristik der typischen Vetarbeitungsverfahren für Rohre kann die Innenfläche der flachen Falten (eingeschränkte Verlängerungszonen) nahezu gerade sein, wie in einigen Abbildungen der WO 2002/002981 A1, z.B. Figur 6, gezeigt. Figur 8D desselben Standes der Technik zeigt sogar eine gerade Innenfläche. Diese flachen Oberflächen bewirken, dass die Werkstoffbeanspruchung in diesen eingeschränkten Verlängerungszonen bei bestimmten relativen Bewegungen der beiden Enden des Rohres in axialer Längsrichtung unabhängig von der Wanddicke ist.

Schließlich verursacht eine gleichmäßige Wanddicke der eingeschränkten Verlängerungszonen im Vergleich zu den anderen Falten hohe Beanspruchungen in diesen eingeschränkten Verlängerungszonen bei der Innendruckbeaufschlagung, da der größte Teil der Last durch diese relativ steiferen Abschnitte aufgenommen wird (siehe WO 2002/002981 A1, Seite 10, Zeilen 6 bis 25: "... wobei diese Ausführung die vorteilhafte Wirkung hat, dass sie eine gleichmäßige Wanddicke in der eingeschränkten Verlängerungssektion des Balgs ermöglicht"). Bei der Innendruckbeufschlagung richtet sich die Beanspruchung im Werkstoff nach dem projizierten druckbeanspruchten Querschnitt und der Wanddicke des Rohres. Anstelle einer gleichmäßigen Wanddicke kann es daher von Vorteil sein, eine stärkere Wanddicke zu haben, insbesondere in den eingeschränkten Verlängerungszonen.

Es besteht daher die Aufgabe der vorliegenden Erfindung darin, ein flexibles Rohr bereitzustellen, das einem hohen Innendruck von bevorzugt 3,2 bar absolut und mehr standhält und darüber hinaus diesem ohne oder nur mit geringem Verlust an Biegeflexibilität standhält.

Die Schwierigkeit dabei ist die erforderliche Kombination von Steifigkeit in axialer Längsrichtung bei der Beaufschlagung mit einem hohen Innendruck wie vorstehend erwähnt (was durch ein gerades Rohr gelöst werden könnte) und der geforderten Biegeflexibilität (was durch ein Rohr mit einem herkömmlichen Faltenbalg gelöst werden könnte). Der Vorteil in Bezug auf die Längssteingkeit eines geraden Rohres ergibt sich zusammen mit einem hohen Biegewiderstand. Die hohe Flexibilität eines Rohres mit einem normal geformten Faltenbalgabschnitt ergibt sich zusammen mit einem geringen Widerstand gegen Längsdehnung bei der Beaufschlagung mit einem hohen Innendruck.

Die Lösung der Aufgabe und somit Gegenstand der vorliegenden Erfindung ist ein flexibles Rohr mit einem Faltenbalg der wenigstens eine, bevorzugt mehrere Falten aufweist, die in der Wand des Rohres ausgebildet sind und über einer Außenfläche des Rohres verlaufen, wobei die Falten in axialer Längsrichtung des Rohres nicht ausdrücklich voneinander beabstandet sind, dadurch gekennzeichnet, dass der Faltenbalg wenigstens eine Falte aufweist, die wiederum zwei einander gegenüberliegende Biegezonen und zwei eingeschränkte Verlängerungszonen aufweist, wobei die eingeschränkten Verlängerungszonen zwischen den einander gegenüberliegenden Biegezonen angeordnet sind und die Höhe der Außenfläche der Falten in den eingeschränkten Verlängerungszonen unter der Höhe der Außenfläche der Falten in den Biegezonen aber noch über dcr Außenfläche des Rohres liegt und die Wanddicke des flexiblen Rohres in mindestens einer eingeschraenkten Verlaengerungszone in mindestens einer Falte, bevorzugt in allen Falten der eingeschränkten Verlängerungszonen des Rohres, gezielt groesser oder kleiner ausgefuehrt als die Wanddicke in den Biegezonen.

Das erfindungsgemäße flexible Rohr mit Faltenbalg erfuellt die Erfordernisse an die Steifigkeit in axialer Längsrichtung bei der Beaufschlagung mit hohem Innendruck und zeigt gleichzeitig eine deutlich verbesserte Biegeflexibilität als ein Kanal mit Fattenbatgkonstruktion gemäß dem Stand der Technik, insbesondere DE 197 07 518 C1 und WO 2002/002981 A1, insbesondere in nicht optimierter Biegerichtung.

Weil die Falten hauptsächlich einer Biegebeanspruchung ausgesetzt sind (es gibt keinen geraden Bereich, wie im Stand der Technik WO 2002/002981 A1 und DE 197 07 518 C1 beschrieben), kann die Flexibilität des Rohres durch gezieltes Erhöhten oder Verringern der Wanddicke der Faltensektionen in den eingeschränkten Verlängerungszonen eingestellt werden. Bei einem geraden Bereich würde eine bestimmte Axialbewegung (Zugbeanspruchung) eine konstante Dehnung im Werkstoff bewirken, unabhängig von der Wanddicke. Ein flexibles Rohr gemäß der vorliegenden Erfindung verursacht bei Druckbeaufschtagung hauptsächlich Biegebeanspruchungen. Im Gegensatz zur reinen Zugbeanspruchung richtet sich die maximale Verformung des mit Biegebeanspruchungen beaufschlagten flexiblen Rohres im Bereich des Faltenbalgs nach der Wanddicke und kann mittels dieser eingestellt werden. Eine Erhöhung der Wanddicke verringert die Beanspruchungen bei der Innendruckbeaufschlagung, hat aber nahezu keinen Einfluss auf die Flexibilität in der optimierten Biegerichtung. Die Biegesteifigkeit in der senkrechten Richtung zur Ebene der eingeschränkten Verlängerungszone wird erhöht, aber aufgrund der vorhandenen Falten ist ein Biegen weiterhin möglich, was ein wesentlicher Vorteil im Vergleich zu bekannten Faltenbalglösungen mit flachen Oberflächen ist. Eine Werkzeugänderung ist für die vorstehend erwähnte über den Umfang partielle Wanddickenanpassung nicht nötig.

Die bevorzugte erfindungsgemaesse Kombination der verringerten Höhen der Außenfläche der Falten in den eingeschränkten Verlängerungszonen und die ueber den Umfang partielle Anhebung der Wanddicke verringern die Beanspruchung des Werkstoffs in dem Faltenbalgabschnitt bei der Druckbeaufschlagung, wobei dies kein oder nur geringen Einfluss auf die Gesamtflexibilität hat.

Eine andere erfindungsgemäße Ausführungsform ist die Kombination der verringerten Höhen der Außenfläche der Falten in den eingeschränkten Verlängerungszonen und die ueber den Umfang partielle Absenkung der Wanddicke zur Verringerung der Beanspruchung des Werkstoffs in dem Faltenbalgabschnitt bei einer Relativbewegung der Rohrenden.

Im ungünstigsten Fall einer Relativbewegung der Rohrenden in axialer Richtung kann das enfindungsgemäße Design auch einer bestimmten Längenzunahme standhalen.

Alle Abschnitte des flexiblen Rohrs werden im Bereich des Faltenbalgs bei Beaufschlagung mit hohem Druck sowohl mit Biege- als auch mit Zugbeanspruchungen beaufschlagt. Weil es keinen geraden Abschnitt in dem beanspruchten Faltenbalgbereich gibt, werden plötzliche Änderungen der Steifigkeit sowie Einkerbungen vermieden, was ebenfalls zur Verhinderung von Spannungskonzentrationen beiträgt und Spitzenbeanspruchungen verringert, welche zum Versagen des Bauteils fuehren koennen.

Beim erfindungsgemäßen flexiblen Rohr liegen die Biegesektionen einander vorzugsweise in einem Winkel von 150-210°, vorzugsweise von 180° gegenüber, und ebenso liegen die eingeschränkten Verlängerungssektionen einander vorzugsweise in einem Winkel von 150-210°, vorzugsweise von 180° gegenüber.

Beim erfindungsgemaessen Rohr nimmt die über den Umfang partielle Anhebung der Wanddicke in den eingeschraenkten Verlaengerungszonen ein vielfaches, maximal ein fuenffaches, der durschnittlichen Wanddicke der Biegezone an. Bevorzugt ist eine Wanddickenanhebung auf 120% bis 300% der dursehnittlichen Wanddicke der Biegezone in den eingeschraenkten Verlängerungszonen.

Bei der Kombination der verringerten Höhen der Außenfläche der Falten in den eingeschränkten Veflängerungszonen und die ueber den Umfang partielle Absenkung der Wanddicke führt bevorzugt eine Wanddickenreduktion auf 35% der durchschnittlichen Wanddicke der Biegezone zu einer vom Lastfall abhängigen, optimierten Eigenschaft des Wellrohres wobei besonders bevorzugt in diesen Faellen Wanddiekenreduktionen auf 50% bis 90% der durchschnittlichen Wanddicke der Biegezone Anwendung finden.

Es gibt keine besondere Begrenzung für das Material, aus dem das erfindungsgemäße flexible Rohr hergestellt wird, vorausgesetzt, dass das Rohr flexibel ist. Wenn blasgeformt, können das flexible Rohr und der Balg aus einem beliebigen thermoplastischen Harz gebildet sein. Der Begriff thermoplastisches Harz" umfasst bevorzugt synthetische Polyamide, Polyester, Polyacetale, Bolckpolyester-Ether-Copolymere, Ethylen-Propylen-Dien-Elastomer (EPDM), Polyolefine wie Polypropylen, sowie Mischungen oder Verschnitte davon. Insbesondere bevorzugt wird Polyamid eingesetzt.

In einer bevorzugten Ausführungsform weist das erfindungsgemäße flexible Rohr zwischen den eingeschränkten Verlängerungszonen und den Biegezonen eine Übergangszone auf. Eine Übergangszone im Sinne der vorliegenden Erfindung beschreibt einen tangentialen, kanten-, und kerbfreien Uebergang von den höheren Falten der Biegezone zu den niedrigeren Falten der eingeschraenkten Veriängerungszone.

In einer bevorzugten Ausführungsform weist ein Faltenbalg eines erfindungsgemäßen flexiblen Rohres wenigstens zwei Falten auf, die wiederum zwei einander gegenüberliegende Biegezonen und zwei eingeschränkte Verlängerungszonen auf, die zwischen den Biegezonen angeordnet sind und wobei die Länge der Falten in der Verlängerungszone in Umfangsrichtung von einer ersten Falte zu einer zweiten Falte hin zunimmt oder abnnimmt. Eine Zunahme oder Abnahme der Länge der Falten in der Verlängerungszone von einer ersten Falte zu einer zweiten Falte hin bedeutet im Sinne der vorliegenden Erfindung, dass sich zwei benachbarte Falten in der Verlaengerungszone in der Laenge in Umfangsrichtung unterscheiden können.

Um große Änderungen der Steifigkeit in dem Faltenbalgabschnitt des erfindungsgemäßen flexiblen Rohres zu vermeiden, die bei relativen Bewegungen der beiden Enden des Rohres Spitzenbeanspruchungen verursachen würden, sind die Übergangszonen zwischen den eingeschränkten Verlängerungszonen und den Biegezonen bevorzugt fließend ohne Kanten oder Kerben geformt. Die in bevorzugter Ausführungsform nicht unterbrochene Form der Außenfläche der nebeneinander befindlichen Falten und auch die nicht unterbrochene Form der jeweiligen Falte selbst sind weitere Gründe für die Vermeidung von Spitzenbeanspruchungen bei den vorstehend genannten Bewegungen und der Innendruckbeanfschlagung.

Zur Verringerung der Längenzunahme bei der Innendruckbeaufschlagung kann in einer bevorzugten Ausführungsform der Außendurchmesser der eingeschränkten Verlängerungszonen weiter verringert werden. Dies hat nur geringen Einfluss auf die Biegeftexibilität des Rohres in der optimierten Biegerichtung. Hierbei ist es wichtig, die Verbindung der Falten fortzuführen, um eine reine Zugbeanspruchung im Faltenbalgbereich während der relativen Bewegungen der beiden Enden des Rohres in axialer Richtung zu verhindern.

Erfindungsgemäß kann die Steifigkeit des Faltenbalgabschnitts in Biegerichtung und auch in Längsdehnungsrichtung durch Anpassung der Wanddicke in den eingeschränkten Verlängerungszonen während des laufenden Herstellungsverfahrens modifiziert werden.

Erfindungsgemäß weist das flexible Rohr der vorliegenden Erfindung wenigstens einen Faltenbalgabschnitt auf. Je nach Aufgabe des flexiblen Rohrs, beispielsweise im Motorraum eines Fahrzeugs, insbesondere Kraftfahrzeugs, kann das Rohr eine Mehrzahl an Faltenbalgabschnitten, bevorzugt 2 bis 10, oder auch eine Vielzahl von Faltenbalgabschnitten, insbesondere 2 bis 100 aufweisen.

Erfindungsgemäß weist ein Faltenbalgabschnitt wenigstens eine Falte auf, bevorzugt eine Vielzahl an Falten, also 2 bis 100, insbesondere bevorzugt eine Mehrzahl, also 2 bis 50 Falten.

Nicht ausdrücklich voneinander beabstandet bedeutet im Sinne der vorliegenden Erfindung, dass der Bereich zwischen zwei nebeneinander liegenden Falten in Axialrichtung nicht glatt ausgebildet sein muss.

Unter der Höhe der Außenfläche der Falte in den Biegezonen bedeutet im Sinne der vorliegenden Erfindung einen geringeren maximalen Aussendurchmesser als der maximale Aussendurchmesser in den Biegezonen.

Über der Außenfläche des Rohres liegend bedeutet im Sinn der vorliegenden Erfindung einen groesseren maximalen Aussendurchmesser als der Aussendurchmesser des glatten Rohres.

Die Falten eines Faltenbalgs sind erfindungsgemäß nebeneinander in axialer Längsrichtung des flexiblen Rohres angeordnet.

In einer bevorzugten Ausführungsform weist die äußere Form zweier miteinander verbundener Falten die Form einer sinusaehnlichen Kurve auf. Dies bedeutet, dass die Falten nicht voneinander beabstandet sind. Wenigstens eine der Falten weist zwei einander gegenüberliegende Biegesektionen und zwei eingeschränkte Verlängerungszonen auf. Die eingeschränkten Verlängerungszonen sind zwischen den beiden Biegezonen angeordnet, und die Höhe der Außenfläche der Falten in der eingeschränkten Verlängerungszone könnte niedriger sein als die Höhe der Außenfläche der Falten in den Biegezonen.

Die vorliegende Erfindung sei anhand der folgenden Figuren erläutert:
Fig. A1 zeigt die Draufsicht eines konventionellen Wellrohrs und Fig. A2 zeigt die entsprechend Seitenansicht eines Wellrohres gemäß dem Stand der Technik.
Fig. B1 zeigt die Draufsicht auf die Biegesektion des erfindungsgemaessen Rohrs. Fig. B2 zeigt die entsprechende Seitenansicht und Fig. B3 die Draufsicht auf die eingescharenkte Verlängerungssektion des erfindungsgemäßen Rohres, wobei der Pfeil die optimierte Beigerichtung anzeigt. Fig. B1, Fig. B2 und Fig. B3 entsprechen der erfindingsgemaessen Darstellung des Rohres ohne Wanddickenanpassung.
Fig. C1 zeigt die Draufsicht auf die Biegesektion des erfindungsgemaessen Rohrs. Fig. C2 zeigt die entsprechende Seitenansicht und Fig. C3 die Draufsicht auf die eingeschraenkte Verlängerungssektion des erfindungsgemäßen Rohres, wobei der Pfeil die optimierte Beigerichtung anzeigt. Fig. C4 zeigt die Schnittansicht des Wellrohrs mit erfindungsgemaess angepasster Wanddicke. Fig. C1 bis C4 entsprechen der erfndingsgemaessen Darstellung des Rohres mit Wanddickenanpassung.
Fig. B1-B3 sowie Fig. C1-C4 zeigen ein erfindungsgemäßes Rohr mit sinusaehnlicher Faltenanordnung. Die Falten sind nicht voneinander beabstandet und es enstehen keine Bereiche zwischen zwei nebeneinander liegenden Falten in Axialrichtung welche glatt ausgebildet sind.

Erfindungsgemäße Rohre werden bevorzugt als Medienleitungen verwendet. Medienleitungen im Sinne der vorliegenden. Erfindung sind bevorzugt Flüssigkeitsleitungen oder Luftleitungen. Bevorzugte Flüssigkeitsleitungen sind Kraüstoftsihläuche, Kühlmittelschläche oder Einfüllstutzen.

Bevorzugte Luftleitungen sind Ansaugleitungen, Kurbelgehäuse-Entlüftungsleitungen, Reinluftleitungen, Ladeluftführungen, Belüftungsrohre, Kühlluftleitungen oder Klimaanlage-Luftleitungen.

Ihr Einsatz erfolgt bevorzugt in Kraftfahrzeugen, insbesondere in deren Motorräumen aber kann auch in allen anderen Anwendungen erfolgen, wo Stoffe unter Druck transportiert werden müssen.

Insbesondere erfolgt der Einsatz eines erfindungsgemäßen flexiblen Rohres für Luft- oder Flüssigkeitsleitungen in Kraftfahrzeugen.

Zu diesem Zweck wird ein erfindungsgemäßes flexibles Rohr bereits während dessen Entwicklungsproresses im Hinblick auf seinen Querschnitt, seinen Durchmesser sowie seiner Außenfläche angepasst. Erfindungsgemaess ist zudem eine weiterfuehrende Anpassung waehrend des Herstellprozesses mittels Wanddickenmodifizierung moeglich.

Bevorzugte Querschnitte eines erfindungsgemäßen flexiblen Rohrs sind kreisförmig, oval oder elliptisch.

Erfindungsgemäße flexible Rohre werden nach bekannten Verfahrensmethoden zum Verarbeiten der oben genannten Einsatzmaterialien hergestellt. Bevorzugte Herstellverfahren sind Blasformen, insbesondere sequenzielles Cooxtrusions-Blasformen, oder Spritzgießverfahren.

### Beispiele

Ein Polymer mit isotropen Werkstofteigenschaften und einem E-Modul von ca. 170 MPa wurde für die Analysen ausgewählt. Drei unterschiedliche Lästige wurden untersucht, und zwar für I) ein herkömmliches, symmetrisches, rundes Rohr mit Faltenbalg gemäß Figur A, II) ein Rohr mit einem erfindungsgemäßen Faltenbalg-Design und derselben Wanddicke wie das Rohr in Hall I gemäß Figur B und III) ein Rohr mit einem erfindungsgemäßen Faltenbalg-Design mit erhöhter Wanddicke in den eingeschränkten Verlängerungszonen gemäß Figur C. Die Wanddicke in den eingeschränkten Verlängerungszonen war von 2,5 mm auf 5 mm erhöht. Der Durchnussquerschnitt betrug bei allen drei Auslegungstallen jeweils 2.316,5 mm².

Der angelegte Druck in Lastfall 1 wurde auf einen Innendruck von 3,5 bar absolut eingestellt; der Lastfall 2 simuliert eine Motorbewegung von 10 mm in der optimierten Biegerichtung, während der Lastfall 3 eine Motorbewegung von 10 mm in der ungünstigsten Biegerichtung simuliert, die senkrecht zur Ebene der eingeschränkten Verlängerungszonen verlief. Alle Lastfälle werden einzeln betrachtet, um Wechselwirkungen zu vermeiden.

Die Ergebnisse in der nachstehenden Tabelle zeigen insbesondere den Unterschied in der resultierenden Längenzunahme aufgrund des Innendrucks und die durch die Motorbewegungen verursachten von-Mises-Spannungen. Dabei ist zu beachten, dass die Biegebeanspruchungen verglichen mit dem Referenz-Faltenbalg-Design I selbst in senkrechter Richtung zur optimierten Biegerichtung bei Design II 25 % höher aber bei Design III aehnlich sind. Des Weiteren ist eine deutliche Reduzierung der Laengenzunahme unter Innendruck bei Design III von 61% erreichbar.

| **Eigenschaft** | **Einheit** | **Herkömmliches Wellrohr (Figur 4)** | **Erfindungsgemäßes flexibles Rohr mit nicht korrigierter Wanddicke** | **Erfindungsgemäßes flexibles Rohr mit korrigierter Wanddlcke** |
|---|---|---|---|---|
| Durchflussquerschnitt | [mm²] | 2.316,5 | 2.316,5 | 2.316,5 |
| Durchschnittliche Wanddicke | [mm] | 2,5 | 2,5 | durchschnittlich 2,5; 5 in eingeschränkten Verlängerungszonen |
| Längenzunahme bei einem Innendruck von 3,5 bar absolut | [mm] | 7,7 | 5,7 | 3 |
| Verringerung der Längenzunahme bei einem innendruck von 3,5 bar absolut gegenüber dem genannten absolut gegenüber dem genannten Referenz-Design | [%] | 0 (Referenz-Design) | -26 | -61 |
| Maximale von-Mises-Spannung bei Motorbewegung in optimierter Biegerichtung | [MPa] | 4 | 4 | 4 |
| Maximale von-Mises-Spannung bei Motorbewegung in senkrechter Richtung zur optimierten Biegerichtung | [MPa] | 4 | 5 | 4 |

## Patentansprüche

1. Flexibles Rohr mit wenigstens einem Faltenbalg, der wenigstens eine, bevorzugt mehrere Falten aufweist, die in der Wand des Rohres ausgebildet sind und über einer Außenfläche des Rohres verlaufen, wobei die Falten in axialer Längsrichtung des Rohres nicht ausdrücklich voneinander beabstandet sind, **dadurch gekennzeichnet, dass** der Faltenhalg wenigstens eine Falte aufweist, die wiederum zwei einander gegenüberliegende Biegezonen und zwei eingeschränkte Vertangerungszonen aufweist, wobei die eingeschränkten Verlängerungszonen zwischen den einander gegenüberliegenden Biegezonen angeordnet sind und die Höhe der Außenfläche der Falten in den eingeschränkten Verlängerungszonen unter der Höhe der Außenfläche der Falten in den Biegezonen aber noch über der Außenfläche des Rohres liegt und die Wanddicke des flexiblen Rohres in mindestens einer eingesehraenkten Verlaengerungszone in mindestens einer Falte, bevorzugt in allen eingeschraenkten Verlaengerungszonen des Rohres, gezielt groesser oder kleiner ausgefuehrt als die Wanddicke in den Biegezonen.

2. Flexibles Rohr nach Anspruch 1, **dadurch gekennzeichnet, dass** dieses zwischen den eingeschränkten Verlängerungszonen und den Biegenzonen eine Übergangszone aufweist.

3. Flexibles Rohr nach Anspruch 1, **dadurch gekennzeichnet, dass** der Faltenbalg wenigstens zwei Falten aufweist, die wiederum zwei einander gegennbertiegende Biegezonen und zwei eingeschränkte Verlängerungszonen aufweisen, die zwischen den Biegezonen angeordnet sind und wobei die Länge der Falten in den Verlängerungszonen in Umfangsrichtung von einer ersten Falte zu einer zweiten Falte hin zunimmt oder abnimmt.

4. Flexibles Rohr nach einem der Ansprüche 1 bis 3, **dadurch gekennzeichnet, dass** dieses aus einem thermoplastischen Harz besteht.

5. Flexibles Rohr gemäß der Ansprüche 1 bis 4, **dadurch gekennzeichnet, dass** dieses einen kreisförmigen, ovalen oder elliptischen Durchmesser aufweist.

6. Verwendung eines flexiblen Rohres gemäß den Ansprüchen 1 bis 5 für Medienleitungen, bevorzugt für Luftleitungen oder Flüssigkeitsleitungen.

7. Verfahren zur Herstellung eines flexiblen Rohres gemäß der Ansprüche 1 bis 5, **dadurch gekennzeichnet, das** dieses nach dem Blasformverfahren oder Spritzgießverfahren hergestellt wird.

8. Verwendung gemäß Anspruch 6, **dadurch gekennzeichnet, dass** es sich im Falle von Flüssigkeitsleitungen um Kühlmittelschläuche, Kraftstoffschläuche oder um Einfüllstutzen handelt.

9. Verwendung gemäß Anspruch 6, **dadurch gekennzeichnet, dass** es sich im Falle von Luftleitungen um Ansaugleitungen, Kurbelgehäuse-Entlüftungsleitungen, Reinluftleitungen, Ladeluftführungen, Belüftungsrohre, Kühlluftelitungen oder Klimaanlage-Luftleitungen handelt.
